**Europäisches Patentamt**

(19) **European Patent Office**

**Office européen des brevets**

(11) Veröffentlichungsnummer: **0 181 354**
**B1**

(12) # EUROPÄISCHE PATENTSCHRIFT

(45) Veröffentlichungstag der Patentschrift:
21.09.88

(51) Int. Cl.⁴: **A 61 F 5/44**

(21) Anmeldenummer: **85901963.0**

(22) Anmeldetag: **14.05.85**

(86) Internationale Anmeldenummer:
**PCT/CH 85/00081**

(87) Internationale Veröffentlichungsnummer:
**WO 85/05264 (05.12.85 Gazette 85/26)**

(54) **URIN-SAMMLER FÜR INKONTINENTE FRAUEN.**

(30) Priorität: **15.05.84 CH 2375/84**

(43) Veröffentlichungstag der Anmeldung:
**21.05.86 Patentblatt 86/21**

(45) Bekanntmachung des Hinweises auf die Patenterteilung:
**21.09.88 Patentblatt 88/38**

(84) Benannte Vertragsstaaten:
**CH DE FR GB LI SE**

(56) Entgegenhaltungen:
**DE-A-1 766 795**
**FR-A-1 408 510**
**GB-A-2 126 902**
**US-A-3 194 238**

(73) Patentinhaber: **NUSSBAUMER, Max,**
**Scheibenstrasse 13, CH- 3014 Bern (CH)**

(72) Erfinder: **NUSSBAUMER, Max, Scheibenstrasse 13,**
**CH- 3014 Bern (CH)**

(74) Vertreter: **Gasser, François W., Postfach 1555, CH-**
**3001 Bern (CH)**

EP 0 181 354 B1

## Beschreibung

Die vorliegende Erfindung betrifft einen Urin-Sammler für inkontinente Frauen gemäss dem Oberbegriff des Patentanspruches 1.

Bei inkontinenten Frauen stellt sich das Problem des Auffangens und Sammelns des unkontrolliert aus der Harnblase und damit der Harnröhre austretenden Urins aus anatomischen Gründen wesentlich anders und viel komplizierter als beim Mann, wo ein Auffangbeutel über das Glied gestülpt werden kann.

Da bei der Frau die Harnröhre zwischen den inneren oder kleinen Schamlippen endet, wo kein Auffangbeutel oder sonstiger Sammler direkt befestigt werden kann, haben sich Frauen bis anhin meistens mit Windeln oder ähnlichem beholfen, da die bekannten Sammler, z. B. nach der JP-A-1 062 936 oder ähnliche, unpraktisch in der Handhabung und/oder unangenehm im Tragen sowie oft undicht sind. Zudem ist es nicht jedermanns Sache, gemass einem anderen bekannten Vorschlag, einen Analstimulator zu tragen, der unter anderem auch den Blasenschliessmuskel derart stimulieren soll, dass die Inkontinenz überwunden wird.

Auch das Urinal für Frauen mit Inkontinenz gemäss der DE-A-1 766 795, bei welchem ein mit einer Schale verbindbarer Urin-Auffangbeutel vorhanden ist, kann nicht befriedigen, da ein in die Harnröhre einzuführender Stift als Verdrehsicherung des Urinals verwendet wird, der sehr störend wirkt. Er ist aber notwendig, um einigermassen sicherzustellen, dass ein rings um den Harnröhrenaustritt herum angeordneter ringförmiger Ansatz, aus dessen Zentrum heraus ein Schlauch abgeht, als Dichtung wirkt.

Der aus der US-A-3 194 238 bekannte Urinsammler mit zwei elastisch federnd ausgebildeten Dichtungsringen oder -wulsten befriedigt ebenfalls nicht, denn auch bei ihm kann der unkontrolliert aus der Harnröhre austretende Urin, insbesondere wenn er über den inneren Dichtungsring, aus dessen Zentrum heraus der Urin in einen Auffangbeutel abgeführt wird, hinaus gelangt, zu einem Gefühl des Nassseins führen und mangels Abflussmöglichkeit in den Auffangbeutel dem Körper entlang entweichen.

Der vorliegenden Erfindung liegt daher die Aufgabe zugrunde, einen Urin-Sammler für inkontinente Frauen vorzuschlagen, der in seiner Handhabung einfach ist, beim andauernden Tragen möglichst wenig stört und in allen Lagen und Situationen ein Maximum an Sicherheit, d. h. Dichtigkeit bietet.

Erfindungsgemäss wird diese Aufgabe durch einen Urin-Sammler gemäss dem Patentanspruch 1 gelöst.

Vorteilhafte Ausführungsvarianten eines erfindungsgemässen Urin-Sammlers werden hiernach an Hand der Zeichnung im Detail beschrieben. Letztere zeigt in

Fig. 1 einen von einer Frau getragenen erfindungsgemässen Urin-Sammler mit den wesentlichsten Zubehören von vorn, in

Fig. 2 dasselbe von schräg hinten, in

Fig. 3 einen Längsschnitt durch einen weiblichen Unterkörper mit getragenem Urin-Sammler, ebenfalls im Längsschnitt, in

Fig. 4 eine perspektivische Ansicht einer weiblichen Scham mit darauf aufzusetzendem Urin-Sammler, ebenfalls perspektivisch, in

Fig. 5 einen Urin-Sammler in der Lage wie er sich am Unterleib getragen präsentiert, perspektivisch von hinten, in

Fig. 6 einen Querschnitt durch eine erste Ausführungsvariante eines erfindungsgemässen Urin-Sammlers, getragen, in

Fig. 7 einen Querschnitt durch eine zweite Ausführungsvariante eines erfindungsgemässen Urin-Sammlers, getragen, in

Fig. 8 einen Längsschnitt durch einen Urin-Sammler gemäss der ersten Ausführungsvariante, in

Fig. 9 die Draufsicht auf den Urin-Sammler nach Fig. 8 in Richtung des Pfeilers IX, in den

Fig. 10 bis 13 Schnitte durch den Urin-Sammler gemäss Fig. 8 entlang den Linien X-X, resp. XI-XI, resp. XII-XII, resp. XIII-XIII, in

Fig. 14 eine teilweise geschnittene perspektivische Ansicht einer dritten Variante eines erfindungsgemässen Urin-Sammlers, in

Fig. 15 eine Draufsicht auf den Urin-Sammler gemäss Fig. 14, in den

Fig. 16 und 17 Längsschnitte durch Details dieser Variante eines Urin-Sammlers, in

Fig. 18 einen Längsschnitt durch den Urin-Sammler gemäss Fig. 15 entlang der Linie XVIII - XVIII, in

Fig. 19 einen Querschnitt durch denselben Urin-Sammler, entlang der Linie XIX - XIX der Fig. 18 und 21, in den

Fig. 20, 21 und 22 Details der Fig. 15, 18 und 19 in grösserem Masstab, in

Fig. 23 eine Unteransicht der dritten Ausführungsvariante eines erfindungsgemässen Urin-Sammlers, wobei ein Teil des Auffangraumes teilweise weggebrochen ist, in

Fig. 24 eine vierte Ausfürungsvariante eines erfindungsgemässen Urin-Sammlers in perspektivischer Sicht, teilweise geschnitten, in

Fig. 25 eine Draufsicht auf diese vierte Ausführungsvariante und in

Fig. 26 einen Längsschnitt durch den Urin-Sammler gemäss Fig. 25, entlang der Linie XXVI - XXVI.

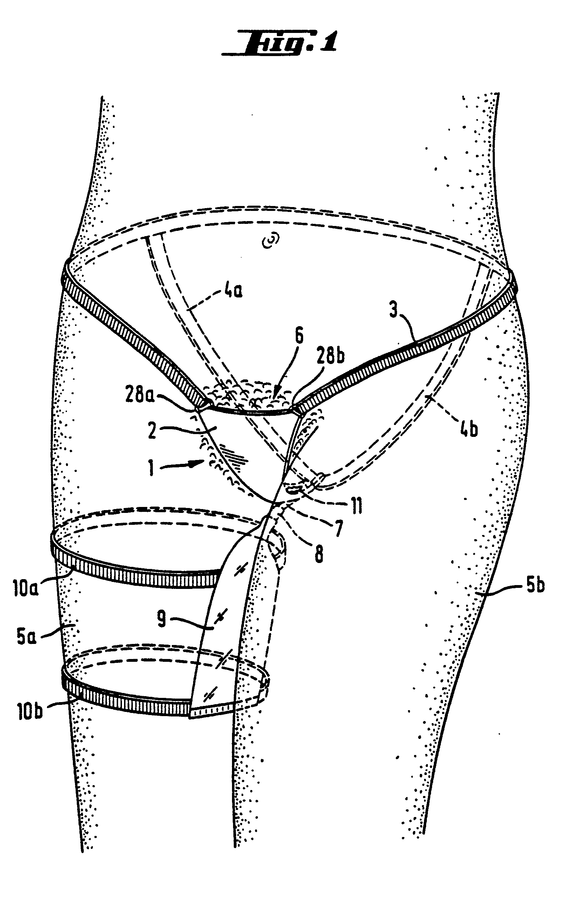

In Fig. 1 ist der erfindungsgemässe Urin-Sammler an einem weiblichen Unterleib getragen in seiner Gebrauchslage dargestellt, wobei Teile davon nur in gestrichelten Linien sichtbar sind, da sie durch den einen Oberschenkel der Frau, resp. deren Unterleib verdeckt sind. Man erkennt in dieser Figur die folgenden allgemeinen Teile des erfindungsgemässen Urin-Sammlers mit den zugehörigen Hilfsmitteln: den generell mit 1 bezeichneten Urin-Sammler und insbesondere dessen Zuschnitt 2, der mit einem Leibgürtel 3 und einem zweiteiligen Hilfsgürtel 4a, 4b in der richtigen Lage zwischen den Oberschenkeln 5a, 5b der den Urin-Sammler tragenden Frau, resp. an deren Scham 6 gehalten wird. Am Zuschnitt 2 erkennt man einen Auslauf 7, der über ein Zwischenstück 8 in einen bekannten Auffang-Beutel 9 mit Schenkelriemen 10a und 10b mündet. Eine sich im Zuschnitt 2 befindliche Kollektor-Struktur 11 ist hier lediglich angedeutet. In dieser Figur ersieht man, dass der erfindungsgemässe Urin-Sammler 1, ähnlich wie ein Bikini-Unterteil getragen wird, wobei der korrekte Sitz des Leibgürtels 3 und des Hilfsgürtels 4a, 4b im wesentlichen die richtige Lage des Zuschnittes 2 mit der darin angeordneten Kollektor-Struktur 11 sichert.

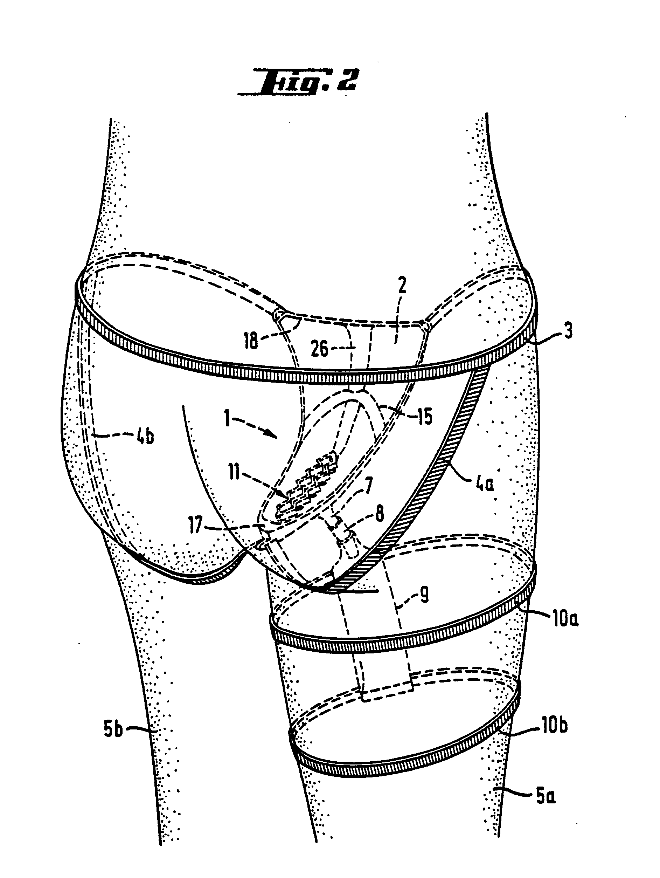

In Fig. 2 ist der Urin-Sammler 1 mit all den in Fig. 1 bezeichneten Hilfsmitteln von der Rückseite der ihn tragenden Frau gezeigt, im wesentlichen zwangsläufig in gestrichelten Linien.

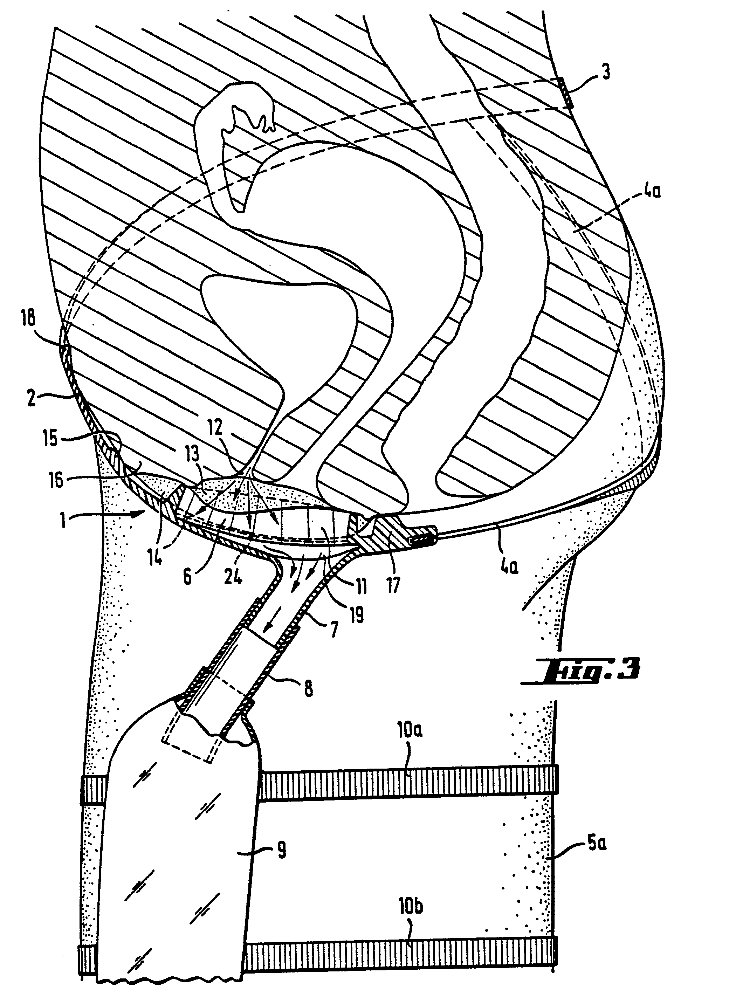

In Fig. 3 ist im Längsschnitt dargestellt, wie der erfindungsgemässe Urin-Sammler 1 im Schitt der Frau liegt, wobei die Lage der Kollektor-Struktur 11 bezüglich des Harnröhrenaustritts 12 und der kleinen und der grossen Schamlippen 13 resp. 14 hier nicht klar ersichtlich ist. Man erkennt hier aber deutlich, wie eine vordere Wulst 15 gegen das Schambein 16 hin den Urin-Sammler 1 gegen vorne abdichtet, wogegen eine hintere Wulst 17 die Abdichtung gegen hinten sicherstellt. Dies, zusätzlich zu der durch die Kollektor-Struktur 11 in Zusammenwirkung mit den Schamlippen 13, 14 erzielten Dichtung. Zudem dient die rund um den Zuschnitt 2 herum laufende Bordure 18 als weitere Dichtung.

Es ist empfehlenswert, nicht nur einen, wie hier dargestellt, einfachen Beutel 9 zu verwenden, sondern einen mit Rückschlagventil, und Auslaufstutzen, um zu verhindern, dass je nach Stellung, die die Frau einnimmt, sich bereits im Beutel 9 befindlicher Urin zurück in den Urin-Sammler 1 fliessen kann und das Entleeren des Beutels 9, ohne ihn abnehmen zu müssen, zu ermöglichen.

Man erkennt in dieser Figur, dass der erfindungsgemässe Urin-Sammler 1 kleinstmöglich gehalten ist und nur den absolut notwendigen Schambereich 6 abdeckt.

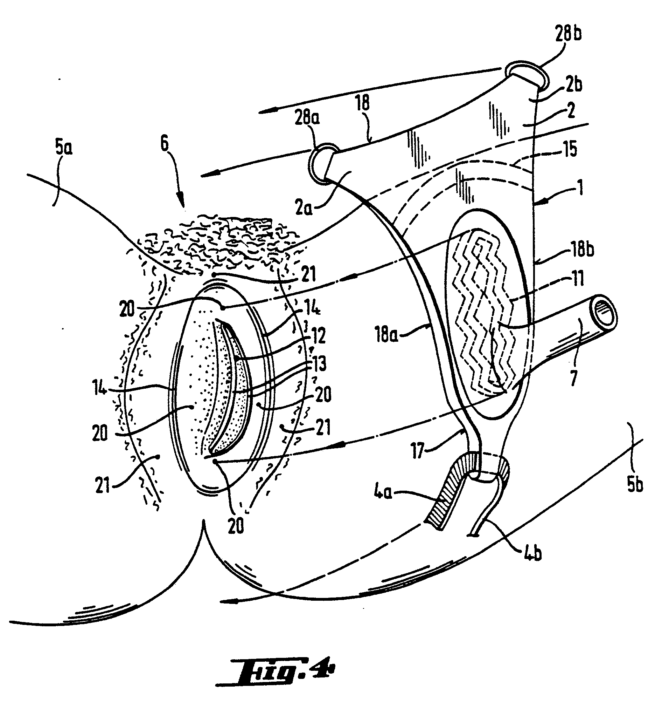

In Fig. 4 ist in einer perspektivischen Explosionszeichnung dargestellt, wohin die einzelnen Teile des erfindungsgemässen Urin-Sammlers 1 am Unterleib der Frau, resp. deren Scham 6 zu liegen kommen. Man erkennt, schematisch dargestellt, die Anordnung der kleinen Schamlippen 13, zwischen welchen die Harnröhre bei 12 austritt, und ausserhalb von welchen sich die grossen Schamlippen 14 befinden, die im Normalfall, nicht wie hier dargestellt, geöffnet sind, sondern über den kleinen Schamlippen 13 liegen.

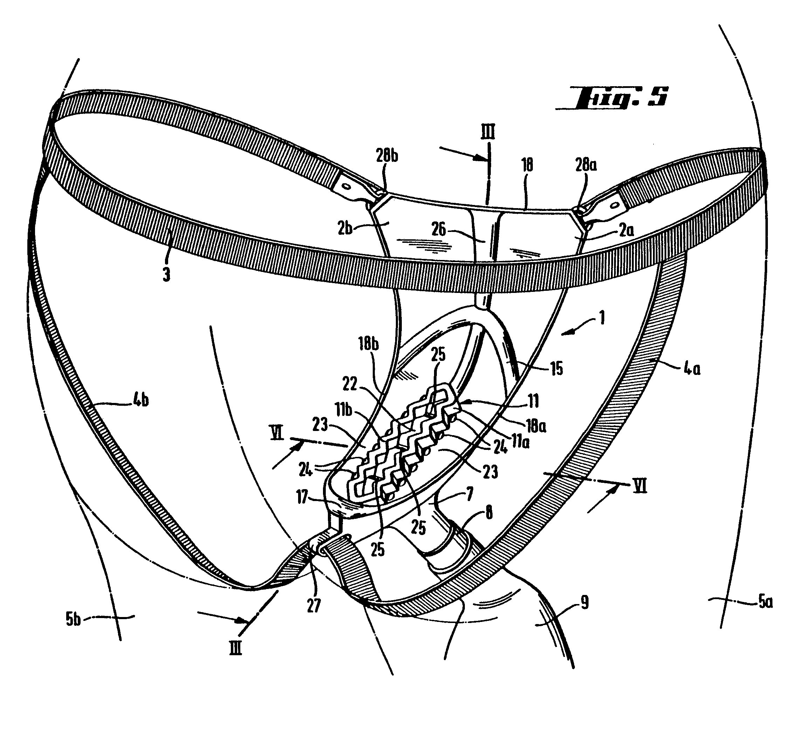

In der Regel wird eine Frau, die nicht übergrosse kleine Schamlippen 13 hat, die hier gezeigte erste Ausführungsvariante des Urin-Sammlers 1 so tragen, dass die Kollektor- Struktur 11, auf deren genaue Ausgestaltung später eingegangen wird, zwischen kleine und grosse Schamlippen 13, resp. 14 in den mit 20 bezeichneten Auflagebereich zu liegen kommt, wie dies auch aus Fig. 6 ersichtlich ist. Derart wird eine innere Zone begrenzt, über die hinaus sich aus dem Harnröhrenaustritt 12 heraus tretender Urin im Normalfall nicht ausbreiten kann, da die Kollektor-Struktur 11 den den inneren Schamlippen 13 entlang fliessenden Urin in den unterhalb der Kollektor-Struktur 11 angeordneten Auffangraum 10 (Fig. 3) ableitet und die Schamlippen 13 und 14 sich eng an die Kollektor-Struktur 11 anlegen. Diese Nasszone 22 (Fig. 5) ist bei allen üblicherweise eingenommenen Stellungen der Frau, sowie bei nicht übertriebenen Bewegungen, soweit dicht, dass Urin nicht aus ihr ausläuft. Sollte dies unerwarteterweise dennoch einmal vorkommen, so sorgen die seitlichen Bereiche 18a und 18b der Bordure, die vorne in die vordere Wulst 17 übergehen, ausserhalb der grossen Schamlippen 14 in dem mit 21 bezeichneten Auflagebereich für eine nochmalige Abdichtung der Schamgegend nach aussen hin. Eventuell in diese äussere Zone, die Feuchtzone 23 (Fig. 5) vordringender Urin wird ebenfalls entlang der Kollektor-Struktur 11 und durch Ablusslöcher 24 (Fig. 5) hindurch in den Auffangraum 19 (Fig. 3) abgeleitet. Aus diesem fliesst der Urin durch den Auslauf 7 aus dem Urin-Sammler 1 heraus, vorteilhafterweise über ein Zwischenstück 8 in einen Beutel 9 (Fig. 3).

In Fig. 5 ist der erfindungsgemässe Urin-Sammler 1 in der getragenen Stellung von hinten gezeigt, wobei der Unterleib der Frau nur angedeutet und die Details der durch den Urin-Sammler 1 abgedeckten und mit ihm Zusammenwirkenden Schamgegend weggelassen sind. Man erkennt in dieser Figur gut die schalenförmige Gestalt, die der Urin-Sammler 1 am Unterleib einnimmt. Gut sichtbar sind auch die Nasszone 22 innerhalb der Kollektor-Struktur 11 und die Feuchtzone 23, die durch die seitlichen Bereiche 18a und 18b der Bordure 18 sowie der vorderen Wulst 15 und der hinteren Wulst 17 begrenzt wird. Gut sichtbar sind in dieser Figur auch Distanzstege 25 zwischen den beiden Seitenteilen 11a und 11b der Kollektor-Struktur 11, deren Aufgabe es ist, zu verhindern, dass die beiden Seitenteile 11a und 11b durch äussere Kräfte, der grossen Schamlippen 14 und/oder der Schenkel 5a, 5b, zusammengedrückt werden, derart, dass der aus der Harnröhre austretende Urin nicht mehr zwischen ihnen abgeleitet werden kann.

In Fig. 6 ist ein Urin-Sammler 1 der ersten Ausführungsvariante in getragener Stellung im Querschnitt im Bereich des Harnröhrenaustrittes 12 dargestellt. Man erkennt dabei den Zuschnitt 2 mit der darin angeordneten Kollektor-Struktur 11, deren beide Seitenteile 11a und 11b mittels der Distanzstege 25 auseinanderge halten werden. Die kleinen Schamlippen 13 liegen hier üblicherweise innerhalb der Kollektor-Struktur 11, wogegen die grossen Schamlippen 14 zwischen dieser und dem aussen mittels der Bordure 18, resp. deren seitlichen Bereichen 18a und 18b verstärkten Zuschnitt 2 liegen. Die Nasszone 22 sowie die Feuchtzone 23 sind durch die im Auflagebereich 20 (Fig. 4) erzielte Dichtung klar voneinander getrennt. Der aus der Harnröhre aus tretende Urin kann ungehindert an den Distanzstegen 25 vorbei entlang der Innenwand der Kollektor-Struktur 11 in den Auffangraum 19 und von da durch den Auslauf 7 abfliessen Urin, der bei unglücklichen Bewegungen oder Stellungen der Frau, oder bei unsorgfältig appliziertem Urin-Sammler 1 in die Feuchtzone 23 gelangt, kann durch die Abflusslöcher 24 ebenfalls in den Auffangraum 19 geleitet werden, von wo er ungehindert abfliessen kann.

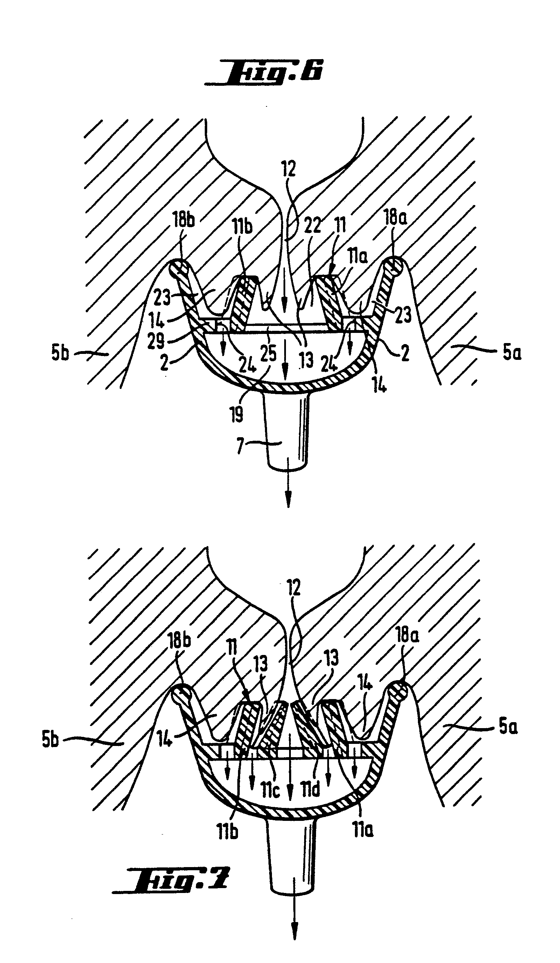

In Fällen, wo durch anatomische Besonderheiten oder erhöhte Bewegungsintensität oder die häufige und langdauernde Einnahme einer liegenden Stellung mittels der hiervor beschriebenen ersten Ausführungsvariante des erfindungsgemässen Urin-Sammlers 1 die Dichtigkeit desselben in Frage gestellt scheint, kann eine zweite Ausführungsvariante gemäss Fig. 7 zur Anwendung gelangen. Diese unterscheidet sich von ersterer nur dadurch, dass sie eine doppelte Kollektor-Struktur 11 aufweist, bei der innerhalb deren Teile 11a und 11b zwei im wesentlichen parallel dazu verlaufende Teile 11c und 11d angeordnet sind. Diese werden zwischen den Kleinen Schamlippen 13 getragen, derart, dass der aus der Harnröhre 12 austetende Urin von ihnen direkt aufgefangen wird.

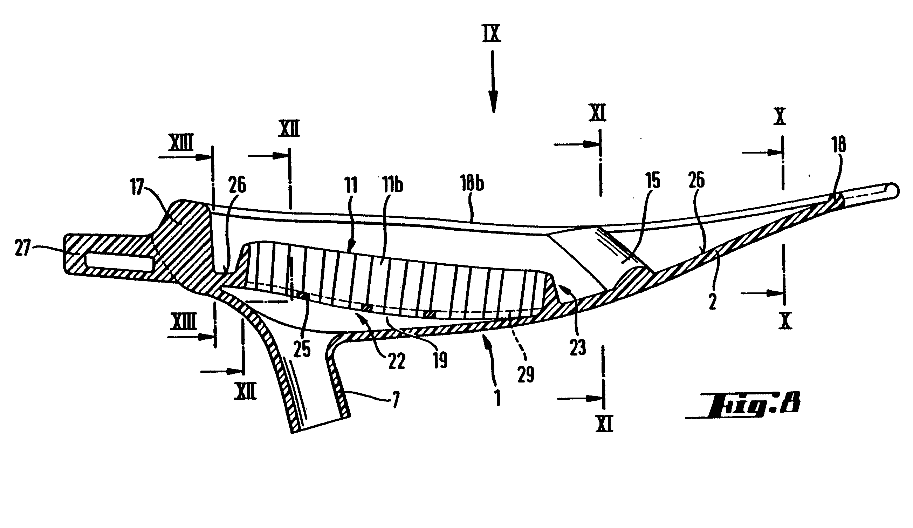

In Fig. 8 ist ein erfindungsgemässer Urin-Sammler 1 für Frauen im Längsschnitt dargestellt. Man erkennt den Zuschnitt 2 mit seiner aussenherum laufenden Bordure 18 als Dichtung und Verstärkung und einem im vorderen Bereich zentral und längs angeordneten Verstärkungsband 26 (Fig. 9). Der Zuschnitt 2 weist innen die Kollektor-Struktur 11 auf, unterhalb von welcher sich der Auffangraum 19 erstreckt, aus welchem der Auslauf 7 herausführt. Die vorne quer durch den Zuschnitt 2 hindurchlaufende vordere Wulst 15, die zusammen mit den seitlichen Bereichen 18a und 18b der Bordure 18 sowie der hinteren Wulst 17 die Feuchtzone 23 begrenzt, ist ebenfalls gut zu erkennen. An letztere anschliessend erkennt man einen Ansatz 27, der der Befestigung resp. dem Durchziehen der hier nicht dargestellten Hilfsgürtel 4a/4b dient.

In Fig. 9 ist der Urin-Sammler 1 nach Fig. 8 aus der Richtung des Pfeiles IX dargestellt. Man erkennt wiederum den Zuschnitt 2, der durch die Bordure 18, 18a, 18b rundum verstärkt ist. Im hinteren, schmaleren Bereich des Zuschnittes 2 ist die Kollektor-Struktur 11, bestehend aus den zwei Seitenteilen 11a und 11b; angeordnet; unter welcher sich der Auffangraum 19 mit dem hier nicht sichtbaren Auslauf 7 befindet. Die beiden Seitenteile 11a und 11b der Kollektor-Struktur 11 sind durch die Distanzstege 25 daran gehindert, zu kollabieren. Hinten laufen die Bordure 18 und die Kollektor-Struktur 11 in die hintere Wulst 17 aus, die den Urinsammler 1 bis auf den Ansatz 27 nach hinten ab schliesst. Im vorderen breiteren Bereich zient sich die vordere Wulst 15 von einem seitlichen Bordurenbereich 18a quer über den Zuschnitt 2 mit seinem längs angeordneten Verstärkungsband 26 bis zum anderen seitlichen Bordurenbereich 18b. Sie bildet den vorderen Abschluss der Feuchtzone 23. An den beiden vorderen Enden 2a und 2b des Zuschnittes 2 ist je ein Ring 28a, resp. 28b angeordnet, an welchen der Leibgürtel 3 (Fig. 1) eingehängt werden kann.

Wie in dieser Figur speziell gut erkennbar ist, besteht die Kollektor-Struktur 11, resp. deren Seitenteile 11a und 11b aus zick-zack-förmigen, vorteilhafterweise nach innen geneigten Erhebungen. Diese Formgebung erinnert an die Oberfläche eines Kaktus und dient wie bei diesem dazu, jede auf sie treffende Feuchtigkeit in die Spitzen der V's zu ziehen, wo sie nach unten abfliessen kann. Dies geschieht bei der Kollektor-Struktur 11 sowohl innen, wo der Urin vom unteren Rand ungehindert in den Auffangraum 19 ablaufen kann, wie aussen, wo gegebenenfalls da auftretender Urin ebenfalls in die Spitzen der V's gezogen und dort nach unten geleitet und damit er sich nicht in der Feuchtzone 23 sammelt, ist vorteilhafterweise in jedem durch die Seitenteile 11a und 11b geformten V ein Abflussloch 24 in der Verbindung 29 angeordnet, derart, dass auch hier ein freier Abfluss des Urins in den darunter liegenden Auffangraum 19 gewährleistet ist.

Vorteilhafterweise wird der Zuschnitt 2 aus leicht flexiblem Material hergestellt, so dass die beim Tragen des Urin-Sammlers auf die Ringe 28a und 28b sowie den Ansatz 27 durch die Gürtel 3 und 4 ausgeübten Zugkräfte dazu führen, dem Zuschnitt 2 eine schalenförmige Gestalt zu geben. Dies führt zu einem besseren Passitz des Urin-Sammlers und damit zu einer besseren Abdichtung desselben, insbesondere in den Auflagebereichen 20 und 21. Aus Komfort- und Hygiene gründen kann der erfindungsgemässe Urin-Sammler ohne weiteres auch mit einer über die Kollektor-Struktur 11 gelegten sterilen Gaze getragen werden, ohne von seiner Wirksamkeit zu verlieren.

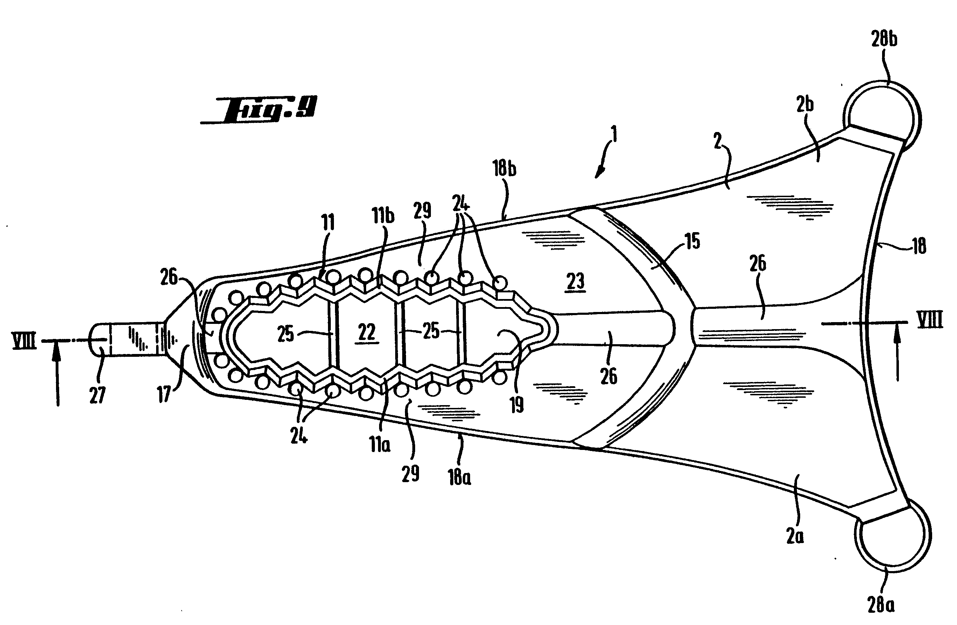

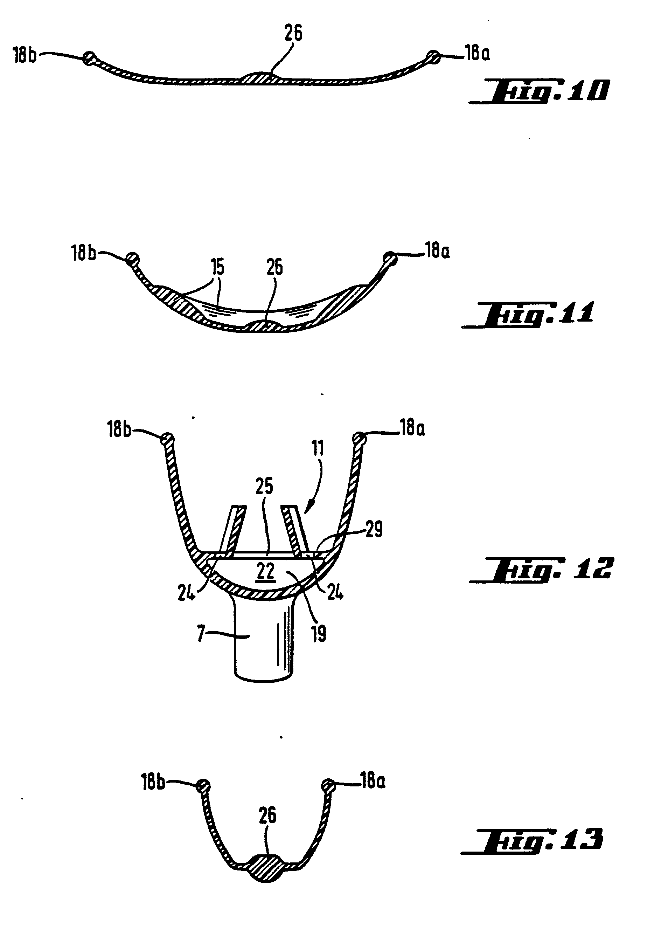

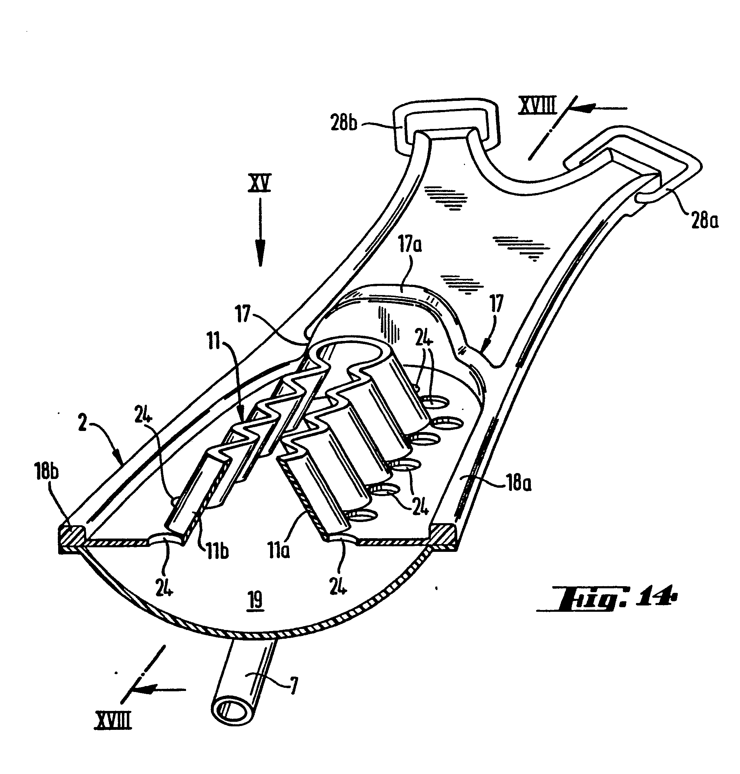

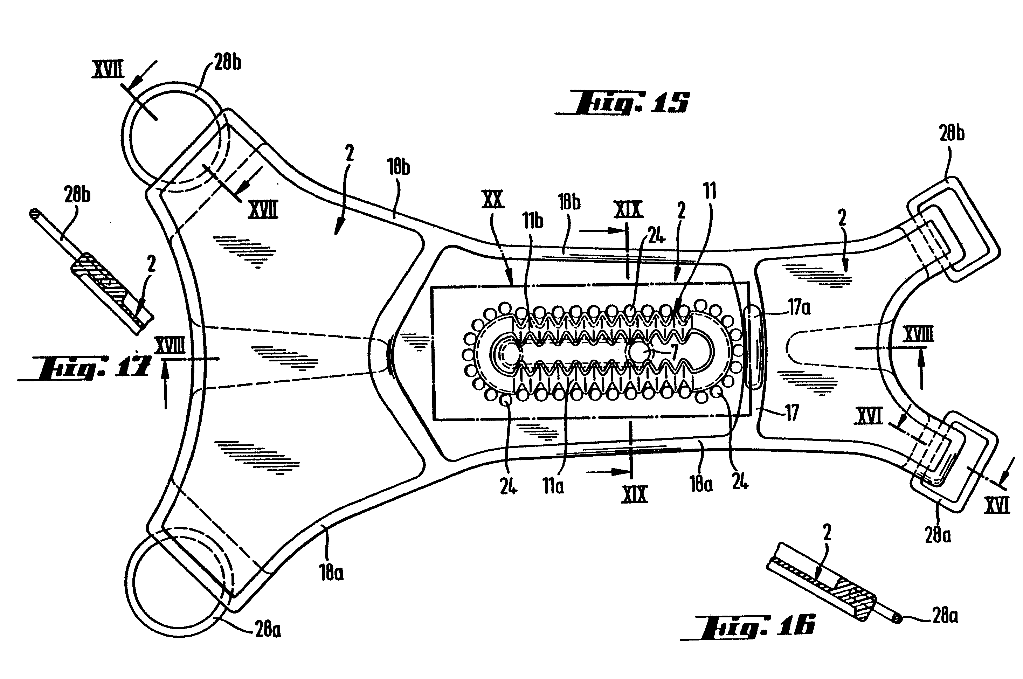

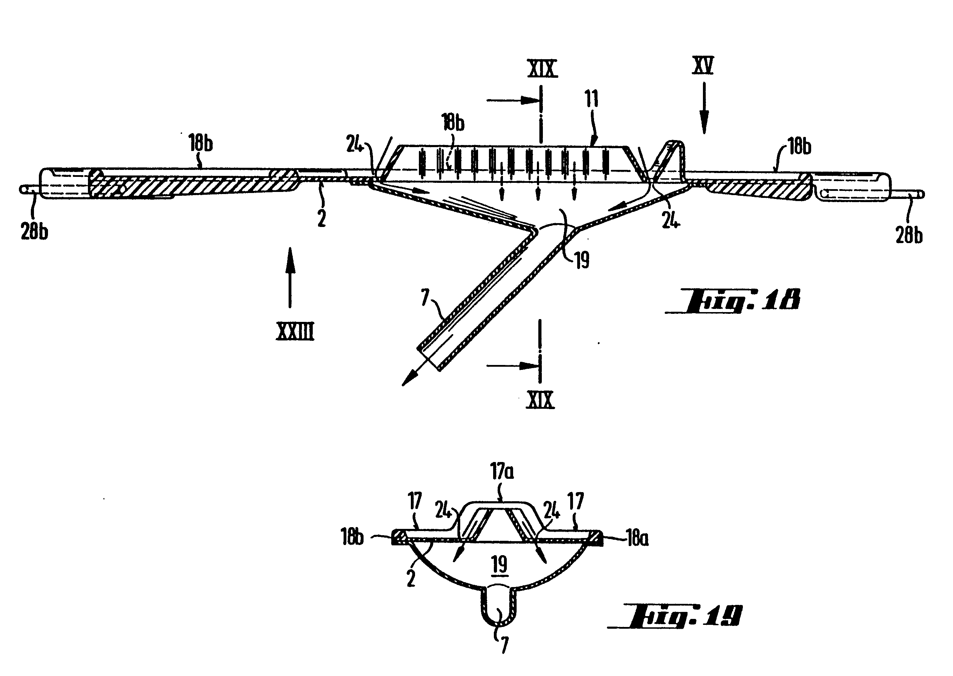

In den Fig. 10 bis 13 sind Querschnitte durch den Urin-Sammler gemäss der Fig. 8 und 9 dargestellt. Diese Figuren bedürfen keiner speziellen Erläuterung.

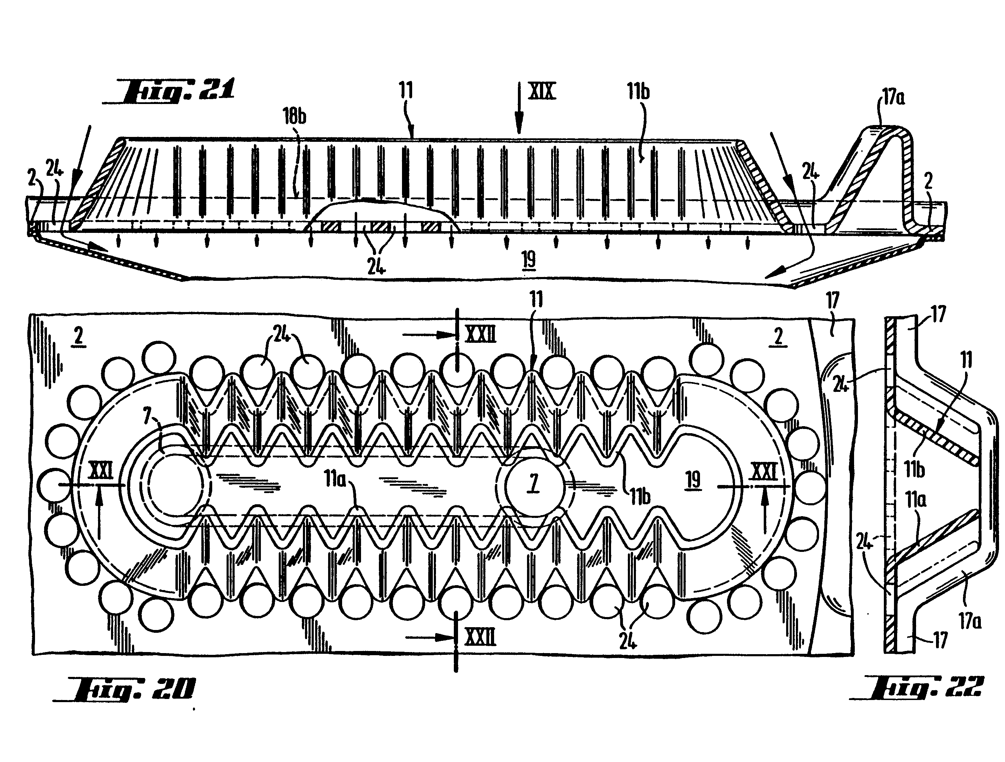

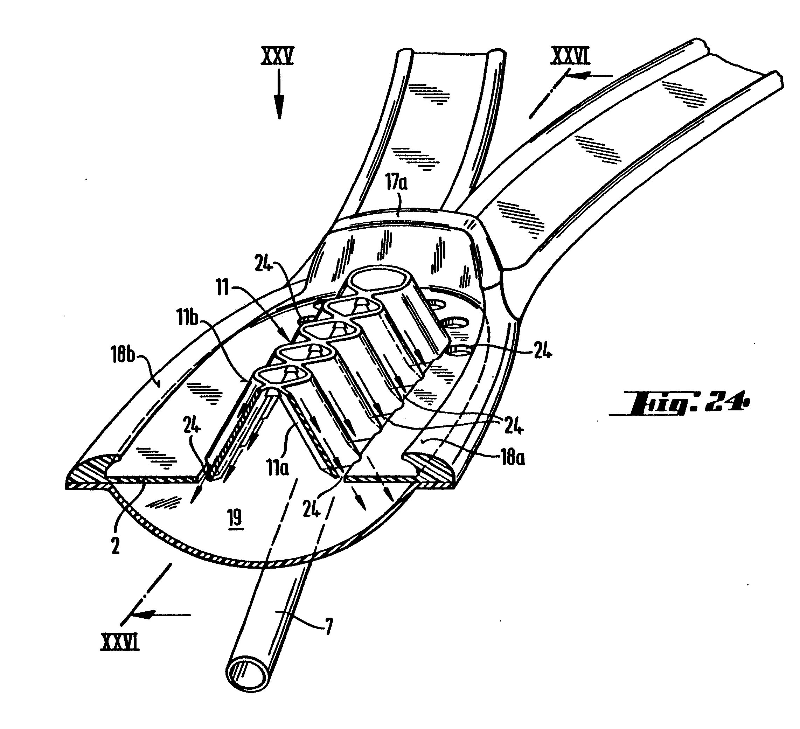

In den Fig. 14 bis 23 ist eine dritte Ausführungsvariante des erfindungsgemässen Urin-Sammlers dargestellt, bei der der Zuschnitt 2 eine leicht andere Form aufweist als in den zwei vorerwähnten Ausführungsvarianten. Zudem ist die hintere Wulst 17 mit einer markanten Erhöhung 17a versehen, um gegen den Gesäss-Spalt hin eine verbesserte Dichtung zu erzielen. Ferner weist dieser Urin-Sammler zwischen den Seitenteilen 11a und 11b der Kollektorstruktur 11 keine Distanzstege auf. Die Kollektorstruktur 11 steht frei über dem Auffangraum 19, aus dem der Auslauf 7 herausführt. Zwecks Erhöhung des Tragkomfortes weist diese Ausführungsvariante des erfindungsgemässen Urin-Sammlers sowohl vorne als auch hinten je zwei Ringe 28a, 28b auf, die, wie sich speziell aus den Fig. 16 und 17 ergibt, in den Zuschnitt 2 eingelassen sind. Es ist empfehlenswert, die hinteren Ringe eckig und die vorderen rund auszugestalten, wie dies in Fig. 15 dargestellt ist.

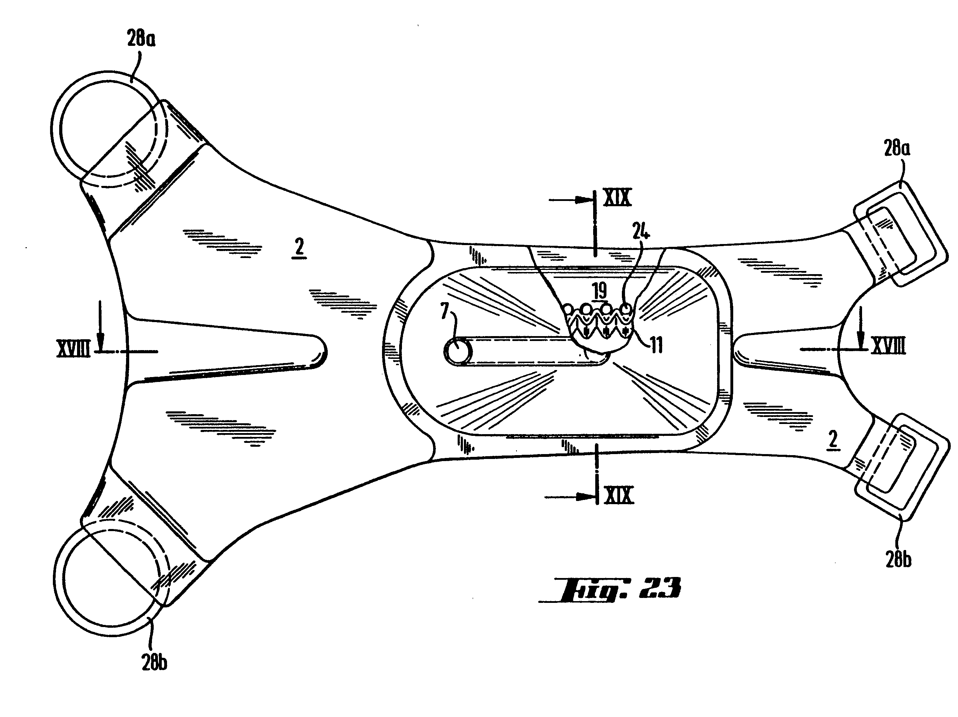

Die Längs- und Querschnitte dieser dritten Ausführungsvariante in den Fig. 18 und 19 bedürfen keiner speziellen Erklärungen. Die Fig. 20, 21 und 22, die den in Fig. 15 innerhalb des strichpunktierten Rechtecks XX liegenden Bereich des erfindungsgemässen Urin-Sammlers in grösserem Maßstab zeigen, veranschaulicht gut, wie die Kollektor-Struktur 11 und die hintere Wulst 17 mit ihrer Erhöhung 17a zusammenwirken. In Fig. 20 ist auch gut ersichtlich, dass bei dieser Ausführungsvariante die Ausflusslöcher 24 vorteilhafterweise rund um die Kollektor-Struktur 11 herum angeordnet sind.

Aus Fig. 23 erkennt man, dass der Auffangraum 19 im wesentlichen aus einer mit dem Zuschnitt 2 an den Bordurenseitenbereichen 18a und 18b sowie den Wulsten 15 und 17 verbundenen Folie gebildet sein kann.

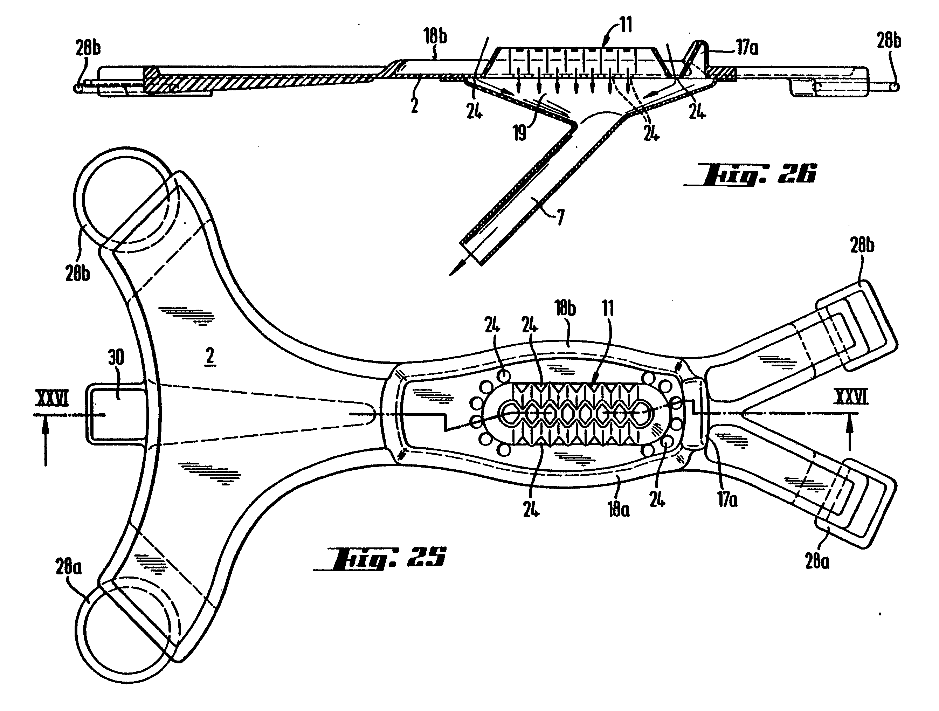

Die Fig. 24 bis 26 zeigen eine vierte Ausführungsvariante des erfindungsgemässen Urin-Sammlers, bei der die Kollektor-Struktur 11, resp. deren Seitenteile 11a und 11b oben, in den Bereichen der nach innen weisenden V's miteinander verbunden sind. Damit können die Distanzstege weggelassen werden, ohne zu riskieren, dass die Kollektor-Struktur 11 durch äussere Kräfte zusammengedrückt werden kann. Diese vierte Ausführungsvariante unterscheidet sich von den drei anderen hiervor beschriebenen ferner noch dadurch, dass der Zuschnitt 2 eine wesentlich kleinere Oberfläche aufweist und hinten sehr weit eingeschnitten ist. Zudem ist die Bordure 18 in ihren seitlichen Bereichen 18a und 18b mit einer Dichtungslippe versehen, was selbstverständlich auch bei den anderen Ausführungsvarianten vorgesehen werden kann. Im Gegensatz zu diesen ist hier ferner vorgesehen, die Abflusslöcher 24 dreieckig zu gestalten, so dass sie sich der V-Form der Kollektorstruktur 11 anpassen. Sofern der Zuschnitt 2 vorne in der Mitte eine Lasche 30 aufweist, kann der Urin-Sammler an Stelle eines Leibgürtels 3 (Fig. 1) auch mit einer Art Hosenträger getragen werden, der vorne ein zwischen den Brüsten liegendes Band aufweist, das sich über die Schultern auftrennt und hinten 2-fach verläuft, um mit den beiden Ringen 28a und 28b verbunden zu werden.

Der Fachmann erkennt leicht, dass der vorliegende erfindungsgemässe Urin-Sammler gegenüber allen bekannten Vorrichtungen für denselben Zweck ganz wesentliche Vorteile aufweist. Er ist möglich klein gehalten und sammelt den unkontrolliert ausfliessenden Harn möglichst nahe an dessen Austrittsstelle. Damit wird derjenige Bereich des weiblichen Unterleibes, der dauernd mehr oder weniger feucht bleibt, praktisch auf die kleinen Schamlippen beschränkt. Dies verhindert weitgehend ein Wundwerden und übermässiges Strapazieren der Haut.

Selbstverständlich kann der hiervor beschriebene Urin-Sammler in Details anders aufgebaut werden, wenn sich dies als sinnvoll erweist. Insbesondere kann eine andere Verbindung mit den Gürteln vorgesehen werden. Ferner kann die Form des Zuschnittes anders gewählt werden, was gegebenenfalls durch die anatomischen Verhältnisse bedingt werden kann. Die Ausdehnung der Kollektor-Struktur und die Grösse des Zuschnittes sind natürlich den Gegebenheiten anzupassen, so dass ein Urin-Sammler für eine junge, schlanke Frau anders aussehen wird als für eine alte, korpulente Frau.

Frauen, die an den Rollstuhl gebunden sind, oder sonst lange sitzen müssen, können vorteilhafterweise ein hufeisenförmiges Kissen verwenden, das vorne offen ist und damit den Ausfluss des Urins aus dem Sammler erleichtert.

Selbstverständlich sind die unterschiedlichen hiervor beschriebenen Ausführungsvarianten des erfindungsgemässen Urin-Sammlers miteinander verbindbar, sodass sich andere, hier nicht erwähnte Kombinationen von Detail-Konstruktionen ergeben können.

**Patentansprüche**

1. Urin-Sammler für inkontinente Frauen, bestehend aus einem Zuschnitt (2) mit Befestigungspunkten (27; 28) für Gürtel (3; 4) und einem Auslauf (7), der mit einem Urinauffangbeutel (9) verbindbar ist, in welchem Zuschnitt (2) eine Kollektor-Struktur (11) angeordnet ist, unter welcher sich ein Auffangraum (19) befindet, aus dem der Auslauf (7) herausführt, wobei im Zuschnitt (2) ferner Mittel (18a, 18b; 15, 17) angeordnet sind, die in Zusammenwirkung mit dem Körper eine um die Kollektor-Struktur (11) herumliegende Feuchtzone (22) begrenzen, über die hinaus kein Urin austreten kann, dadurch gekennzeichnet, dass die Kollektorstruktur (11) zwei vorne und hinten zusammenlaufende Seitenteile (11a; 11b) umfasst, die in Draufsicht zick-zack-förmig sind.

2. Urin-Sammler nach Anspruch 1, dadurch gekennzeichnet, dass die Mittel aus den seitlichen Teilen (18a, 18b) einer Bordure (18) des Zuschnittes (2) bestehen, die vorne und hinten in Wulste (15; 17) übergehen.

3. Urin-Sammler nach Anspruch 1, dadurch gekennzeichnet, dass die Seitenteile (11a; 11b) der Kollektor-Struktur (11) auf ihrer Aussenseite mit dem Zuschnitt (2) verbunden sind, wobei unten an jedem V in der Verbindung ein Abflussloch (24) angeordnet ist, durch die Urin in den Auffangraum (19) abfliessen kann.

4. Urin-Sammler nach Anspruch 1, dadurch gekennzeichnet, dass innerhalb der zwei Seitenteile (11a, 11b) zwei weitere Seitenteile (11c, 11d) mit zick-zack-förmigem Grundriss angeordnet sind.

**Claims**

1. Urine collector for incontinent women, consisting of a section (2) having fastening points (27; 28) for belts (3; 4) and an outlet (7), which can be connected to a urine collection bag (9), in which section (2) a collection structure (11) is disposed, under which there is located a collecting area (19), from which the outlet (7) leads, in the section (2) there also being disposed means (18a, 18b; 15, 17), which in conjunction with the body limit a wet zone (22) surrounding the collection structure (11), via which no urine can escape, characterised in that the collection structure (11) comprises two lateral parts (11a; 11b) converging at the front and at the back, which are zig-zag shape in plan view.

2. Urine collector according to claim 1, characterised in that the means consist of the lateral parts (18a, 18b) of a border (18) of section (2), which at the front and at the back change into reinforcements (15; 17).

3. Urine collector according to claim 1, characterised in that the lateral parts (11a; 11b) of the collection structure (11) are connected on their exterior to section (2), there being disposed at the bottom of each V in the connection a drain hole (24), through which urine can flow into the collecting area (19).

4. Urine collector according to claim 1, characterised in that inside the two lateral parts (11a, 11b) are disposed two further lateral parts (11c, 11d) having a zig-zag shaped cross-section.

**Revendications**

1. Appareil collecteur d'urine pour femmes incontinentes, comportant une pièce découpée en forme (2), pourvue de points d'attache (27; 28) pour une ceinture (3; 4) et d'un orifice d'évacuation (7) qui peut être relié à un sac (9) porté par le sujet pour recueillir l'urine, la pièce de forme (2) étant garnie d'une structure de recueil (11) sous laquelle est ménagée une cavité de recueil (19) communiquant avec l'orifice d'évacuation (7), la pièce de forme (2) étant en outre pourvue de moyens d'étanchéité (18a, 18b; 15, 17) adaptés à coopérer avec le corps du sujet, pour délimiter un zone mouillée (22) entourant la structure de recueil (11), en empêchant toute fuite d'urine hors de cette zone; caractérisé en ce que la structure de recueil (11) comprend deux parties latérales (11a; 11b) ayant en plan une forme en zig-zag.

2. Appareil conforme à la revendication 1, caractérisé en ce que les moyens d'étanchéité sont constitués par les parties latérales (18a, 18b) d'une bordure (18) de la pièce de forme, ces parties latérales se raccordant à un bourrelet antérieur (15) et à un bourrelet postérieur (17).

3. Appareil conforme à la revendication 1, caractérisé en ce que les parties latérales (11a; 11b) de la structure recueil (11) sont reliées à la pièce de forme (2) à l'endroit de leur bord extérieur, un trou d'écoulement (24) étant prévu en-dessous de chaque V de la zone de liaison, pour permettre à l'urine de passer dans la cavité de recueil (19).

4. Appareil conforme à la revendication 1, caractérisé en ce qu'il comporte, à l'intérieur des deux parties latérales (11a, 11b), deux autres parties latérales (11c, 11d) ayant en plan une forme en zig-zag.

# *Fig. 1*

_Fig. 2_

**Fig. 3**

**Fig. 4**

**Fig. 5**

0 181 354

*Fig:6*

*Fig:7*

Fig. 8

*Fig.9*

0 181 354

Fig. 10

Fig. 11

Fig. 12

Fig. 13

*Fig. 14.*

Fig. 15

Fig. 16

Fig. 17

**Fig. 18**

**Fig. 19**

Fig. 21

Fig. 22

Fig. 20

Fig. 23

Fig. 24.

_Fig. 26_

_Fig. 25_

0 181 354